(19) Europäisches Patentamt
European Patent Office
Office européen des brevets

(11) Veröffentlichungsnummer: **0 012 371**
**B1**

(12) # EUROPÄISCHE PATENTSCHRIFT

(45) Veröffentlichungstag der Patentschrift: **22.12.82**

(51) Int. Cl.³: **C 07 D 233/10**, C 07 D 233/06

(21) Anmeldenummer: **79104961.2**

(22) Anmeldetag: **06.12.79**

(54) **Verfahren zur Herstellung von 2-Imidazolinen.**

(30) Priorität: **16.12.78 DE 2854428**

(43) Veröffentlichungstag der Anmeldung: **25.06.80 Patentblatt 80/13**

(45) Bekanntmachung des Hinweises auf die Patenterteilung: **22.12.82 Patentblatt 82/51**

(84) Benannte Vertragsstaaten: **BE CH DE FR GB IT**

(56) Entgegenhaltungen: **CHEMICAL ABSTRACTS, Band 86, Nr. 19, 09-03-1977, Seiten 566, Zusammenfassung 140048x. Columbus, Ohio, USA**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Dockner, Toni, Dr. Dipl.-Chem., Grossgasse 6, D-6701 Meckenheim (DE)**
Erfinder: **Kempe, Uwe, Dr. Dipl.-Chem., Carl-Bosch-Strasse 44, D-6703 Limburgerhof (DE)**
Erfinder: **Krug, Herbert, Mussbacher Strasse 49, D-6700 Ludwigshafen (DE)**
Erfinder: **Magnussen, Peter, Dr., Professor-Dillinger-Weg 25, D-6702 Bad Duerkheim 1 (DE)**
Erfinder: **Praetorius, Werner, Dr. Dipl.-Chem., Rossdoerfer Strasse 75, D-6100 Darmstadt (DE)**
Erfinder: **Szymanski, Hans Joachim, Mutterstadter Strasse 17, D-6707 Schifferstadt (DE)**

EP 0 012 371 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 2-Imidazolinen

Die Erfindung betrifft ein neues Verfahren zur Herstellung von 2-Imidazolinen durch Umsetzung von 1,2-Diaminen mit Nitrilen oder Carbonsäuren bzw. Carbonsäurederivaten in einem Verhältnis von 1 bis 2 Mol Ausgangsstoff (II) je Mol Ausgangsstoff (III) oder (IV), in der Gasphase, in Gegenwart von Trägergasen, die unter den Reaktionsbedingungen inert sind, vorteilhaft Stickstoff, bei einer Temperatur von 300 bis 380 °C in Gegenwart von γ-Aluminiumoxid und/oder Siliciumdioxid als Katalysator mit einem Porenradius von 15 bis 100 Å und einer spezifischen Gesamtoberfläche von 250 bis 500 Quadratmetern pro Gramm, gegebenenfalls in Gegenwart von Phosphorsäure, in der Wirbelschicht während einer Verweilzeit von 2 bis 40 Sekunden.

Aus J. Chem. Soc. (1947), Seiten 497 bis 505 ist es bekannt, dass Salze des Äthylendiamins durch Umsetzung mit Nitrilen bei 200 bis 270 °C 2-substituierte 2-Imidazoline liefern. Die Ausbeuten an Endstoff sind unbefriedigend. Die japanische Patentveröffentlichung 24 965/1964 gibt an, dass Schwefel in Stücken die Kondensation von 1,2-Diaminen mit Nitrilen katalysiert. Es wird darauf hingewiesen und an einigen Katalysatoren auch gezeigt, dass mit anderen Schwefelverbindungen als Schwefel in Stücken die Ausbeute niedriger liegt oder es überhaupt zu keiner Umsetzung kommt. Schwefelwasserstoff zeigt so nur 16,1 Prozent Ausbeute, und im Falle von Octylmercaptan tritt keine Umsetzung ein. Anorganische Sulfide oder Polysulfide werden nicht erwähnt. Das Verfahren ist mit Bezug auf Einfachheit des Betriebs und Ausbeute an Endstoff nicht befriedigend.

Es ist aus den Chemischen Berichten, Band 74, Seiten 1763 bis 1766 (1941) bekannt, dass man Laurinsäure mit einer überschüssigen Menge von Äthylendiamin und Äthylendiaminhydrochlorid bei einer Temperatur von 280 bis 290 °C zu Undecylimidazolin-hydrochlorid umsetzt. Es wird beschrieben, dass die Umsetzung nur geringe Mengen Imidazolin neben dunkelgefärbten Zersetzungsprodukten und viel Dilauroyläthylendiamin liefert, wenn man statt des salzsauren Salzes nur die freie Base verwendet. Aus den Reaktionsgemischen muss der Endstoff durch mehrere Reinigungsstufen, z.B. Lösen des Gemischs in Wasser, Fällung mit Natronlauge, Ausschütteln mit Äther, Waschen der Ätherextrakte mit Kochsalzlösung und Abdampfen des Äthers, isoliert werden.

Eine Arbeit aus Journal of the American Chemical Society, Band 70, Seiten 1629 bis 1632 (1948) lehrt, dass man die Umsetzung auch mit der freien Base und in Abwesenheit von Salzsäure bei einer Temperatur von 180 bis 220 °C durchführen kann; ein Molverhältnis der Reaktionspartner von 1:1 und die Gegenwart von Benzol im Reaktionsgemisch werden, wie Erläuterungen und alle Beispiele zeigen, für diese Umsetzung vorgeschrieben. Während der Umsetzung wird ein azeotropes Gemisch von Benzol und Wasser abdestilliert. Trotz dieser besonderen Verfahrensweise werden neben dem Imidazolin stets grössere Mengen eines hochsiedenden Nebenproduktes gefunden. Es wird beschrieben und durch die Beispiele gezeigt, dass die Ausbeute an diesem Nebenprodukt mit der Zahl der Kohlenstoffatome im Alkancarbonsäuremolekül steigt; so zeigen beispielsweise Umsetzungen mit Essigsäure 32,7 Prozent und mit Myristinsäure 92,5 Prozent Ausbeute an hochsiedendem Nebenprodukt. Im Falle der Stearinsäure erhält man praktisch quantitativ nur das Nebenprodukt. Entsprechend betragen die Ausbeuten an Imidazolin im Falle des Einsatzes von Essigsäure 26,6 Prozent, von Ameisensäure 13,3 Prozent, von Propionsäure 12,9 Prozent und von Capronsäure 15,4 Prozent. Die Ausbeuten bei längerkettigen Carbonsäuren sind nicht angegeben. Aus dem Nebenprodukt kann nur auf mühseligem Wege, z.B. durch Behandlung mit wässriger Natronlauge, Extraktion mit Äther, Trocknen des Äthers über festem Kaliumhydroxid, Abdampfen des Äthers und fraktionierte Destillation, ein Teil des Imidazolins zurückgewonnen werden.

Es ist aus der DE-A-2 512 513 ein Verfahren zur Herstellung von 2-Imidazolinen durch Umsetzung von 1,2-Diaminen mit Nitrilen in Gegenwart von Polysulfiden als Katalysatoren bekannt. Nachteile des Verfahrens sind die Bildung von schwefelhaltigen, schwer abtrennbaren Nebenprodukten, die den Katalysator bei der folgenden Dehydrierreaktion beeinträchtigen.

Die DE-A-2 615 886 beschreibt ein Verfahren zur Herstellung von 2-Imidazolinen durch Umsetzung von Alkancarbonsäuren mit einem Überschuss von 1,2-Diaminen bei Temperaturen unterhalb 160 °C unter Abtrennung von Wasser. Nachteile des Verfahrens sind die geringen Raum-Zeit-Ausbeuten; man benötigt mindestens 2 Mol Diamin je Mol Alkancarbonsäure. Die russische Patentschrift SU-A-533 593 (Kurzreferat; CA. 86, 19, Seite 566 (140048X)) behandelt nur Fettsäuren, aus deren Verhalten der Fachmann keine Schlüsse auf das Verhalten von kurzkettigen erfindungsgemässen Ausgangsstoffen III bzw. IV, insbesondere auch auf das der nichtaliphatischen Ausgangsstoffe ziehen kann. Weiterhin wird das Verfahren bei tieferer Temperatur in flüssiger Phase durchgeführt.

Es wurde nun gefunden, dass man 2-Imidazoline der Formel (I)

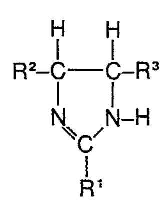

(I)

worin $R^1$ jeweils einen Alkylrest mit 1 bis 7 Kohlenstoffatomen, einen Aralkylrest mit 7 bis 12 Kohlenstoffatomen oder einen Phenylrest bedeutet, $R^2$

einen Alkylrest mit 1 bis 7 Kohlenstoffatomen oder ein Wasserstoffatom bedeutet, durch katalytische Umsetzung von 1,2-Diaminen mit Carbonylverbindungen oder Nitrilen, vorteilhaft erhält, wenn man 1,2-Diamide der Formel (II)

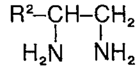 (II),

worin $R^2$ die vorgenannte Bedeutung hat,
a) mit Nitrilen der Formel (III)

$$R^1\text{-}CN \quad (III)$$

oder
b) mit Carbonsäuren bzw. Carbonsäurederivaten der Formel (IV)

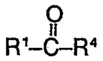 (IV),

worin $R^1$ die vorgenannte Bedeutung besitzt und $R^4$ den Rest $-OR^2$, $-H_2$ oder 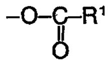 bezeichnet, worin $R^1$ und $R^2$ die vorgenannten Bedeutungen besitzen, in einem Verhältnis von 1 bis 2 Mol Ausgangsstoff (II) je Mol Ausgangsstoff (III) oder (IV), in der Gasphase, in Gegenwart von Trägergasen, die unter den Reaktionsbedingungen inert sind, vorteilhaft Stickstoff, bei einer Temperatur von 300 bis 380 °C in Gegenwart von γ-Aluminiumoxid und/oder Siliciumdioxid als Katalysator mit einem Porenradius von 15 bis 100 Å und einer spezifischen Gesamtoberfläche von 250 bis 500 Quadratmetern pro Gramm, gegebenenfalls in Gegenwart von Phosphorsäure, in der Wirbelschicht während einer Verweilzeit von 2 bis 40 Sekunden umsetzt.

Die Umsetzung kann für den Fall der Verwendung von Äthylendiamin und Acetonitril bzw. von 1,2-Diaminopropan und Isobuttersäure durch die folgenden Formeln wiedergegeben werden:

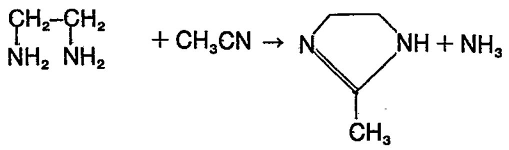

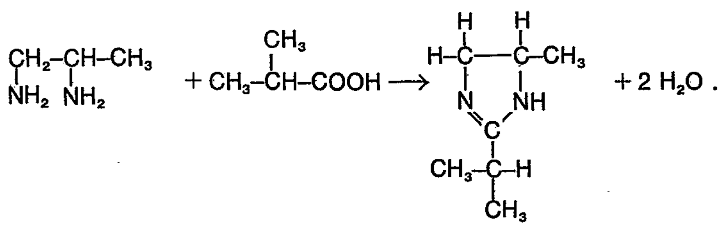

Im Vergleich zu den erstgenannten bekannten Verfahren liefert das Verfahren nach der Erfindung auf einfacherem und wirtschaftlicherem Wege 2-Imidazoline in besserer Ausbeute und Reinheit. Obwohl mit freier Base umgesetzt wird, werden Zersetzungsprodukte, Komplexverbindungen oder hochsiedende Nebenprodukte nicht in wesentlichem Masse beobachtet. Umständliche, mehrstufige Reinigungsoperationen werden vermieden.

Im Vergleich zu den in den deutschen Offenlegungsschriften 2 512 513 und 2 615 886 beschriebenen Verfahren liefert das Verfahren nach der Erfindung überraschend auf einfacherem und wirtschaftlicherem Wege 2-Imidazoline in teilweise besserer Ausbeute und Reinheit und besserer Raum-Zeit-Ausbeute. Im Hinblick auf die DE-A-2 615 886 benötigt man geringere Mengen an Diamin. Schwefelhaltige Katalysatoren werden nicht verwendet, das Verfahren ist umweltfreundlicher. Der erfindungsgemäss verwendete Katalysator hat vergleichsweise zu dem in der DE-A-2 512 513 beschriebenen Polysulfidkatalysator eine höhere Lebensdauer und liefert den Endstoff in gleichbleibend hoher Ausbeute auch bei einem Betrieb der Anlage über mehr als 3000 Stunden. Auch nach der Regenerierung liegt die Lebensdauer des erfindungsgemässen Katalysators höher, z.B. wird der Endstoff auch noch bei einem Betrieb über mehr als 1000 Stunden in hoher Ausbeute hergestellt. Die Bildung von Crackprodukten auf dem Katalysator, die die aktiven Zentren des Katalysators verringern bzw. inhibieren, wird in wesentlichem Masse vermindert. Die Wirtschaftlichkeit und Betriebssicherheit der Herstellung von 2-Imidazolinen wird auf die genannte Weise entscheidend verbessert. Diese vorteilhaften Ergebnisse sind im Hinblick auf den Stand der Technik auch gegenüber beispielsweise SU-A-533 593 überraschend.

Die Ausgangsstoffe werden miteinander in einem Verhältnis von 1 bis 2, insbesondere 1 bis 1,2 Mol Ausgangsstoff (II) je Mol Ausgangsstoff (III) oder (IV), umgesetzt.

Werden als Ausgangsverbindungen (IV) Ester oder Säuren verwendet, so können die Reste $R^2$ im Ausgangsstoff (II) und (IV) gleich oder verschieden sein. Es kommen auch gemischte Säureanhydride als Ausgangsstoffe (IV) in Betracht.

Als Ausgangsstoffe (II) kommen z.B. in Frage: Äthylendiamin, 1,2-Propylendiamin, 1,2-Butylendiamin, 1,2-Pentylendiamin, 1,2-n-Hexylendiamin, 1,2-n-Heptylendiamin, 1,2-n-Octylendiamin, 1,2-n-Nonylendiamin.

Als Ausgangsstoffe (III) kommen z.B. in Betracht: Acetonitril, Propionsäurenitril, Buttersäurenitril, Isobuttersäurenitril, Pentancarbonsäurenitril, Caprylsäurenitril, Trimethylessigsäurenitril, Isovaleriansäurenitril, Valeriansäurenitril, Benzoesäurenitril, Phenylpropionsäurenitril, Phenylessigsäurenitril.

Als Ausgangsstoffe (IV) kommen z.B. in Betracht: Ameisensäure, Essigsäure, Propionsäure, Buttersäure, Isobuttersäure, Capronsäure, Önanthsäure, α-Äthylbuttersäure, 2-Methylbutansäure, Trimethylessigsäure, Valeriansäure, Isovaleriansäure, Isocapronsäure, oder entsprechende Gemische wie die bei der Herstellung von natürlichen oder synthetischen Fettsäuren erhaltenen Gemische. Solche Gemische fallen z.B. durch Fettspaltung, durch Paraffinoxidation oder durch die Oxosynthese aus Olefinen, Kohlenoxid und Wasser, an; den vorgenannten Säuren entsprechende Säureamide, Säureanhydride und Ester mit Methanol, Isopropanol, Äthanol, n-Propanol, tert.-Butanol, sek.-Butanol, n-Hexanol, n-Butanol, iso-Butanol, n-Pentanol, n-Heptanol.

Die Umsetzung wird bei einer Temperatur von 300 bis 380 °C, drucklos oder unter Druck, zweckmässig 1 bis 10 bar, kontinuierlich oder diskontinuierlich durchgeführt, Temperatur- und Druckbedingungen werden so gewählt, dass die Umsetzung in der Gasphase stattfindet. Trägergase, die unter Reaktionsbedingungen inert sind wie Stickstoff, werden mitverwendet, zweckmässig in einer Menge von 20 bis 80 Gewichtsprozent, bezogen auf die Gewichtsmenge Ausgangsstoff (II).

Die Umsetzung wird in Gegenwart von Siliciumdioxid und/oder γ-Aluminiumoxid durchgeführt. Die genannten Verbindungen kommen allein oder im Gemisch miteinander in Betracht. Bei der kontinuierlichen Verfahrensweise verwendet man in der Regel 5 bis 15 Mol Ausgangsstoff II pro Stunde und Liter Katalysator.

Vorteilhaft stellt man den $SiO_2$-Katalysator wie folgt her: Handelsübliches Natronwasserglas wird unter Zusatz von Schwefelsäure über die Zwischenstufe eines Kieselsäure-Sols in ein wasserreiches Kieselsäure-Hydrogel überführt, das mit wässrigem Ammoniak (20 Gew.-%) eluiert und so von Salzen befreit wird. Das so behandelte Hydrogel wird mit der Phosphorsäure, vorteilhaft unter Zusatz von Oxalsäure, z.B. von 5 bis 15 Gewichtsprozent Oxalsäure, bezogen auf Siliciumdioxid, in einer Mühle oder einer anderen, Scherkräfte erzeugenden Vorrichtung behandelt, wobei Peptisation eintritt. Die erhaltene wässrige Phase wird in einem Gasstrom von 200 bis 400 °C, z.B. im Rauchgasstrom, versprüht, wobei der Katalysator in Gestalt eines körnigen Pulvers anfällt. Diese Form der Katalysatorherstellung ist gerade bei Verwendung im Wirbelschichtverfahren vorteilhaft. Die Phosphorsäure wird zweckmässig in Gestalt einer wässrigen Lösung, mit 50 bis 90, vorzugsweise mit 60 bis 80 Gewichtsprozent Phosphorpentoxid auf die Kieselsäureverbindung aufgetragen.

Formgebung und Grösse der Katalysatorteilchen sind für die Umsetzung nicht von ausschlaggebender Bedeutung.

Als spezifische Gesamtoberfläche wird die gesamte innere und äussere Oberfläche des Katalysators, bezogen auf 1 Gramm Katalysator, verstanden. Zur Bestimmung der spezifischen Gesamtoberfläche können die üblichen Methoden zur Bestimmung der Gesamtoberfläche von Katalysatoren, z.B. die BET-Methode (Ullmanns Encyklopädie der technischen Chemie, Band 9, Seite 266), herangezogen werden. Bei dem erfindungsgemässen Wirbelschichtverfahren sind Korngrössen von 0,06 bis 0,5 Millimeter vorteilhaft. Der Porenradius des Katalysators liegt bei 15 bis 100, vorteilhaft bei 30 bis 80 Å. Die Form kann beliebig, z.B. amorph, strang-, kugelförmig oder körnig, gewählt werden. Bezüglich der Herstellung der Katalysatoren wird auf Houben-Weyl, Methoden der Organischen Chemie, Band 4/2, Seiten 142 ff und Ullmanns Encyklopädie der technischen Chemie, Band 9, Seiten 271 ff und Band 15, Seiten 712 ff verwiesen.

Als Zusatzkatalysator wird gegebenenfalls Phosphorsäure verwendet, vorteilhaft in einer Menge von 0,01 bis 1, insbesondere 0,01 bis 0,1 Gewichtsprozent, bezogen auf Ausgangsstoff (II). Zweckmässig sind eine Menge von 1 bis 30, insbesondere 5 bis 15 Gewichtsprozent Phosphorsäure je Gewichtsmenge γ-Aluminiumoxid und/oder Siliciumdioxid. Die Phosphorsäure kann ganz oder teilweise als Phosphorpentoxid, Orthophosphorsäure, Metaphosphorsäure, Pyrophosphorsäure oder Polyphosphorsäure, z.B. von 72 bis 88 Gewichtsprozent $P_2O_5$, vorliegen und wird hier als $H_3PO_4$ berechnet, unabhängig von der tatsächlichen Konstitution der Phosphorsäure bzw. des Phosphorsäureanhydrids. Der Zusatzkatalysator kann in üblicher Weise, z.B. durch Tränken, Sprühen, Vermischen, gemeinsames Vermahlen, mit dem Metalloxid/Metallphosphat-Katalysator vereinigt werden. Vorteilhafter setzt man den Zusatzkatalysator vor Beginn der Reaktion dem Ausgangsstoff (II) oder dem Ausgangsgemisch aus den Ausgangsstoffen und gegebenenfalls dem Trägergas zu.

Die Reaktion kann wie folgt durchgeführt werden: Ein Gemisch der Ausgangsstoffe (II) und (III) wird über den auf die Reaktionstemperatur erhitzten Katalysator in einem Wirbelschichtreaktor geleitet. Ausser dem Gemisch werden noch unter den Reaktionsbedingungen inerte Gase, z.B. Stickstoff, zugeführt werden. Die Verweilzeit beträgt von 2 bis 40, insbesondere 3 bis 20 Sekunden im Reaktionsraum. Aus dem den Reaktor verlassenden Reaktionsgemisch wird der Endstoff in üblicher Weise, z.B. durch fraktionierte Destillation, isoliert. Man kann aber auch dem Reaktionsgemisch Proben entnehmen, durch analytische, z.B. gaschromatographische Bestimmung des Verhältnisses von Endstoff (I) und Ausgangsstoff (II) im Reaktionsgemisch den Umsatz feststellen und das Reaktionsgemisch ohne Abtrennung des Endstoffs direkt weiterverarbeiten, z.B. zu den entsprechenden Imidazolen.

Der Katalysator bzw. Katalysator auf Trägern wird durch Inertgas oder einem Gemisch von Ausgangsstoff II und III bzw. IV und Inertgas als Wirbelschichtgas bei Normaldruck oder vermindertem oder erhöhtem Druck in einer Wirbelschicht gehalten. Entsprechend kann die Gesamtmenge

oder eine Teilmenge an Ausgangsstoffen getrennt von dem Wirbelschichtgas in den Wirbelschichtreaktor eingeleitet werden. Die Ausgangsstoffe können auch in einem beheizten Vorratsgefäss flüssig gehalten und in einen Verdampfer, der dem Wirbelschichtreaktor vorgeschaltet ist, dosiert werden. Man leitet vorteilhaft einen schwachen Stickstoffstrom, zweckmässig von 5000 bis 50 000 Volumenteilen Stickstoff je Stunde, durch den Verdampfer. Die verdampften Ausgangsstoffe werden zusammen mit dem Stickstoffstrom durch das Katalysatorbett geleitet. Die Konzentration des Ausgangsstoffs (II) im Inertgas beträgt vorteilhaft 0,1 bis 50 Volumenprozent. Man kann das Verfahren nach der Erfindung in einem einfachen oder unterteilten, offenen oder geschlossenen Wirbelschichtsystem mit und ohne Fliessstaubzirkulation durchführen. Bezüglich Reaktoren, Durchführung, Verfahrensvarianten und Reaktionsbedingungen des Wirbelschichtverfahrens wird auf Ullmanns Encyklopädie der technischen Chemie, Band 1, Seiten 916 ff, verwiesen. Die Aufarbeitung des Reaktionsgemisches erfolgt in vorgenannter Weise.

Die nach dem Verfahren der Erfindung herstellbaren 2-Imidazoline (I) sind wertvolle Ausgangsstoffe für die Herstellung von Farbstoffen, Pflanzenschutzmitteln und Pharmaceutica. 2-Imidazoline (I) werden als Katalysatoren für Polymerisationsreaktionen und Aldolkondensationen eingesetzt. Durch Dehydrierung an Aluminium/Zinkoxidkatalysatoren liefern sie die entsprechenden Imidazole. Bezüglich der Verwendung wird auf die vorgenannten Veröffentlichungen und Ullmanns Encyklopädie der technischen Chemie, Band 13, Seiten 331 und 338 verwiesen.

Die in den folgenden Beispielen aufgeführten Teile bedeuten Gewichtsteile. Die Gewichtsteile verhalten sich zu den Volumenteilen wie Kilogramm zu Liter.

Beispiel 1

132 Teile 1,2-Diaminoäthan werden stündlich unter Rühren und Kühlung mit 120 Teilen Essigsäure bei 38 °C gemischt. Das gebildete Äthylendiamin-Monoacetat erstarrt unterhalb 35 °C zu einer kristallinen Masse, oberhalb dieser Temperatur bleibt es flüssig. 252 Teile dieses Gemisches werden pro Stunde aus einem Vorratsgefäss in einen auf 300 °C erhitzten horizontalen Quarzverdampfer dosiert, und der Dampf zusammen mit 5000 Volumenteilen pro Stunde Stickstoff durch den auf 300 °C erhitzten Wirbelreaktor geleitet. Der Wirbelreaktor ist ein vertikal auf dem Verdampfer sitzendes, elektrisch beheiztes Quarzrohr, das nach unten mit einer eingeschmolzenen Quarzfritte abgeschlossen ist. Das Quarzrohr ist mit 200 Teilen eines Katalysators aus 5 Gewichtsprozent $H_3PO_4$ und 95 Gewichtsprozent γ-Aluminiumoxid zur Hälfte gefüllt. Die Verweilzeit in der Katalysatorzone im Wirbelzustand beträgt 3,5 Sekunden. Die Höhe der Katalysatorzone beträgt im Wirbelzustand 80 mm. Die den Reaktor verlassenden Dämpfe werden kondensiert und fraktioniert destilliert. Man erhält stündlich 159,6 Teile (95% der Theorie, bezogen auf Essigsäure) 2-Methylimidazolin vom Kp 73 °C (2,66 mbar), Fp 103 °C. Der Umsatz beträgt 88,5 Prozent, bezogen auf Diamin II. Die Ausbeute blieb auch noch nach 300 Stunden Betrieb konstant.

Beispiel 2

Man verfährt wie in Beispiel 1, setzt jedoch als Katalysator nur γ-$Al_2O_3$ ohne $H_3PO_4$ ein. Man erhält 161 Teile (96% der Theorie) 2-Methylimidazolin vom Kp 73 °C (2,66 mbar).

Beispiel 3

Man verfährt wie in Beispiel 1, setzt jedoch einen Katalysator mit 95 Gewichtsprozent $SiO_2$ und 5 Gewichtsprozent $H_3PO_4$ ein. Man erhält 159,6 Teile (95% der Theorie) 2-Methylimidazolin vom Kp 73 °C (2,66 mbar).

Beispiel 4

Man verfährt wie in Beispiel 1, setzt jedoch einen reinen $SiO_2$-Katalysator ohne $H_3PO_4$ ein. Man erhält 159,6 Teile (95% der Theorie) 2-Methylimidazolin vom Kp 73 °C (2,66 mbar).

Beispiel 5

176 Teile n-Buttersäure und 132 Teile Äthylendiamin werden mit 40 000 Volumenteilen $N_2$ in den Quarzverdampfer eingeführt, bei 300 °C verdampft und bei gleicher Temperatur über 200 Gewichtsteile des in Beispiel 1 beschriebenen Katalysators geführt. Man erhält 215 Teile 2-n-Propylimidazolin (96% der Theorie, bezogen auf eingesetzte n-Buttersäure) vom Kp 87 °C (5,32 mbar).

Beispiel 6

Man verfährt wie in Beispiel 5, setzt jedoch 288 Teile n-Octansäure und 132 Teile Äthylendiamin ein. Man erhält 319 Teile 2-n-Heptylimidazolin (95% der Theorie, bezogen auf eingesetzte Octansäure vom Kp 133 °C (5,32 mbar).

Beispiel 7

Man verfährt wie in Beispiel 5, setzt jedoch 176 Teile Isobuttersäure mit 162,8 Teilen 1,2-Diaminopropan ein. Man erhält 201 Teile 2-Isopropyl-4,(5)-methylimidazolin (80% der Theorie, bezogen auf eingesetzte Isobuttersäure) vom Kp 105 bis 106 °C (32 mbar).

Beispiel 8

Man verfährt wie in Beispiel 5, setzt jedoch 272 Teile Benzoesäuremethylester mit 132 Teilen Äthylendiamin ein. Man erhält 277 Teile 2-Phenylimidazolin (95% der Theorie, bezogen auf eingesetzten Benzoesäuremethylester) vom Kp 295 °C (1013 mbar).

Beispiel 9

Man verfährt wie in Beispiel 5, setzt jedoch 300 Teile Phenylessigsäuremethylester mit 132 Teilen Äthylendiamin ein. Man erhält 288 Teile Benzylimidazolin (90% der Theorie, bezogen auf eingesetzten Phenylessigsäuremethylester) vom Kp 155 bis 161 °C (3 mbar).

Beispiel 10
Man verfährt wie in Beispiel 5, setzt jedoch 82 Teile Acetonitril mit 132 Teilen Ätylendiamin ein. Man erhält 149 Teile (91% der Theorie) 2-Methylimidazolin vom Kp 73 °C (2,66 mbar).

Beispiel 11
Man verfährt wie in Beispiel 5, setzt jedoch 206 Teile Benzonitril mit 132 Teilen Äthylendiamin ein. Man erhält 273,6 Teile (95% der Theorie) 2-Phenylimidazolin vom Kp 295 °C (1013 mbar).

Beispiel 12
Man verfährt wie in Beispiel 5, setzt jedoch 118 Teile Acetamid mit 132 Teilen Äthylendiamin ein. Man erhält 159,6 Teile (95% der Theorie) 2-Methylimidazolin vom Kp 73 °C (2,66 mbar).

**Patentanspruch**

Verfahren zur Herstellung von 2-Imidazolinen der Formel (I)

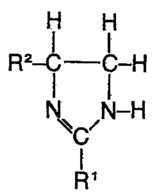 (I)

worin $R^1$ jeweils einen Alkylrest mit 1 bis 7 Kohlenstoffatomen, einen Aralkylrest mit 7 bis 12 Kohlenstoffatomen oder einen Phenylrest bedeutet, $R^2$ einen Alkylrest mit 1 bis 7 Kohlenstoffatomen oder ein Wasserstoffatom bedeutet, durch katalytische Umsetzung von 1,2-Diaminen mit Carbonylverbindungen oder Nitrilen, dadurch gekennzeichnet, dass man 1,2-Diamine der Formel (II)

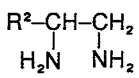 (II),

worin $R^2$ die vorgenannte Bedeutung hat,
a) mit Nitrilen der Formel (III)

$R^1$–CN (III)

oder
b) mit Carbonsäuren bzw. Carbonsäurederivaten der Formel (IV)

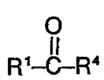 (IV),

worin $R^1$ die vorgenannte Bedeutung besitzt und $R^4$ den Rest $-OR^2$, $-NH_2$ oder 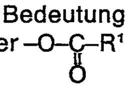 bezeichnet, worin $R^1$ und $R^2$ die vorgenannten Bedeutungen besitzen, in einem Verhältnis von 1 bis 2 Mol Ausgangsstoff (II) je Mol Ausgangsstoff (III) oder (IV), in der Gasphase, in Gegenwart von Trägergasen, die unter den Reaktionsbedingungen inert sind, vorteilhaft Stickstoff, bei einer Temperatur von 300 bis 380 °C in Gegenwart von γ-Aluminiumoxid und/oder Siliciumdioxid als Katalysator mit einem Porenradius von 15 bis 100 Å und einer spezifischen Gesamtoberfläche von 250 bis 500 Quadratmetern pro Gramm, gegebenenfalls in Gegenwart von Phosphorsäure, in der Wirbelschicht während einer Verweilzeit von 2 bis 40 Sekunden umsetzt.

**Claim**

A process for the preparation of a 2-imidazoline of the formula

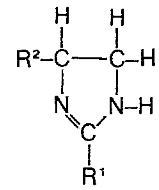 (I)

where $R^1$ is alkyl of 1 to 7 carbon atoms, aralkyl of 7 to 12 carbon atoms or phenyl, and $R^2$ is alkyl of 1 to 7 carbon atoms or hydrogen, by catalytic reaction of a 1,2-diamine with a carbonyl compound or a nitrile, wherein a 1,2-diamine of the formula

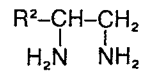 (II),

where $R^2$ has the above meaning, is reacted
a) with a nitrile of the formula

$R^1$–CN (III)

or

b) with a carboxylic acid or carboxylic acid derivative of the formula

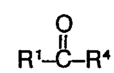 (IV)

where $R^1$ has the above meaning and $R^4$ is $-OR^2$, $-NH^2$ or 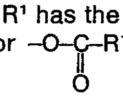, $R^1$ and $R^2$ having the above meanings, in a ratio of 1 to 2 moles of starting material II per mole of starting material III or IV, in the gas phase in the presence of a carrier gas which is inert under the reaction conditions, advantageously nitrogen, at from 300 to 380 °C in the presence of γ-aluminium oxide and/or in the presence of silicon dioxide as catalyst having a pore radius of from 15 to 100 Å and a specific total surface area of from 250 to 500 $m^2/g$, in the presence or absence of phosphoric acid, in a fluidized bed, the residence time being from 2 to 40 seconds.

**Revendication**

Procédé pour la préparation de 2-imidazolines de formule

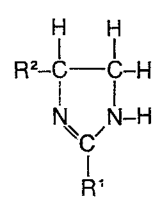 (I)

dans laquelle $R^1$ représente chaque fois un radical alcoyle à 1–7 atomes de carbone, un radical aralcoyle à 7–12 atomes de carbone ou un radical phényle, $R^2$ représente un radical alcoyle à 1–7 atomes de carbone ou un atome d'hydrogène, par réaction catalytique de 1,2-diamines avec des composés carbonylés ou des nitriles, caractérisé en ce qu'on fait réagir des 1,2-diamines de formule

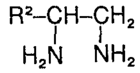 (II),

dans laquelle $R^2$ a la signification donnée ci-dessus,

a) avec des nitriles de formule

$R^1$–CN (III)

b) ou avec des acides carboxyliques ou des dérivés d'acides carboxyliques de formule

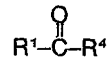 (IV)

dans lesquelles $R^1$ a la signification donnée ci-dessus et $R^4$ désigne le radical $-OR^2$, $-NH_2$ ou 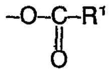 ($R^1$ et $R^2$ ayant les significations données ci-dessus, dans un rapport de 1 à 2 moles de substance de départ (II) par mole de substance de départ (III) ou (IV), en phase gazeuse, en présence de gaz porteurs qui sont inertes dans les conditions de la réaction, de préférence l'azote, à une température de 300 à 380 °C, en présence d'alumine $\gamma$ et/ou de silice servant de catalyseur et ayant un rayon de pores de 15 à 100 Å et une surface spécifique totale de 250 à 500 $m^2/g$, le cas échéant en présence d'acide phosphorique, en couche turbulente pendant une durée de séjour de 2 à 40 secondes.